(19) **Europäisches Patentamt / European Patent Office / Office européen des brevets**

(11) **EP 4 736 869 A2**

(12) **EUROPEAN PATENT APPLICATION**

(43) Date of publication:
06.05.2026 Bulletin 2026/19

(51) International Patent Classification (IPC):
*A61K 38/53* $^{(2006.01)}$

(21) Application number: 26157809.0

(22) Date of filing: 23.02.2022

(52) Cooperative Patent Classification (CPC):
(C-Sets available)
**A23L 33/17; A23L 33/18; A61K 38/1796;
A61K 38/44; A61K 38/446; A61K 38/53;
A61P 3/06; A61P 3/10; A61P 19/10; A61P 25/28;
C12Y 111/01006; C12Y 115/01001;
C12Y 603/01002;** A23V 2002/00        (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  23.02.2021  KR 20210024072

(62) Document number(s) of the earlier application(s) in
accordance with Art. 76 EPC:
**22760057.4 / 4 298 924**

(71) Applicant: **Industry-Academic Cooperation
Foundation
Gyeongsang National University
Jinju-si, Gyeongsangnam-do 52828 (KR)**

(72) Inventors:
• **KIM, Hyun Joon
52696 Jinju-si (KR)**
• **KIM, Young Bum
21505 Incheon (KR)**
• **KANG, Jae Soon
52718 Jinju-si (KR)**
• **JUNG, Soonwoong
52723 Jinju-si (KR)**
• **SONG, Miyoung
52697 Jinju-si (KR)**
• **BAEK, Ji Hyeong
52718 Jinju-si (KR)**
• **PARK, Sang Won
52718 Jinju-si (KR)**
• **KIM, Hwajin
52718 Jinju-si (KR)**
• **YOO, Dae Young
52650 Jinju-si (KR)**

(74) Representative: **Ipsilon
12 Avenue d'Italie
75013 Paris (FR)**

Remarks:
•The complete document including Reference
Table(s) and the Sequence Listing(s) can be
downloaded from the EPO website
•This application was filed on 11-02-2026 as a
divisional application to the application mentioned
under INID code 62.

(54) **COMPOSITION FOR PREVENTING, AMELIORATING, OR TREATING DISEASE CAUSED BY PROTEIN NITRATION COMPRISING PEPTIDE WITH TERMINAL TYROSINE AS EFFECTIVE COMPONENT**

(57)    According to the present invention, the peptide with terminal tyrosine has an excellent effect on inhibiting protein nitration and also an excellent effect on preventing, ameliorating, or treating disease symptoms in chronic immobilization stress-induced depression/cognitive impairment model, Alzheimer's disease model, epileptic seizure model, stroke model, type 2 diabetes model, acute renal failure model, or hyperammonemia model.

**EP 4 736 869 A2**

(52) Cooperative Patent Classification (CPC): (Cont.)

C-Sets
A23V 2002/00, A23V 2200/322, A23V 2200/328,
A23V 2200/332

**Description**

TECHNICAL FIELD

The present invention relates to a composition for preventing, ameliorating, or treating a disease caused by protein nitration comprising a peptide with terminal tyrosine as effective component.

BACKGROUND ART

Oxidative stress is known to occur when oxygen generated during cellular metabolism acts as free radicals, causing damage to cells and leading to various diseases. In particular, when reactive oxygen species (ROS) or reactive nitrogen species (RNS) present within cells lead to the generation of peroxynitrite (ONOO⁻) at tyrosine included in a protein, protein nitration is caused. Protein nitration is considered a phenomenon accompanied in oxidative processes, and when specific proteins undergo nitration, their structure can be altered, leading to a decrease in activity or loss of normal function, ultimately contributing to the development of various diseases. Protein nitration has been reported to be closely related to signal transductions in cell, diseases caused by inflammatory response, neurodegenerative diseases, aging, and others, and also recently has been implicated in conditions such as asthma, diabetes, cancer, chronic stress-induced depression, type 2 diabetes, and acute renal diseases. Therefore, research on protein nitration plays a crucial role in both biological and clinical contexts, and there is a need for studies focused on developing substances that can inhibit the protein nitration.

Meanwhile, Korean Patent Registration No. 1897400 discloses a "Composition for inhibiting the activity of tyrosine decarboxylase and a method for producing fermented food using the same", and Korean Patent Publication No. 2018-0021746 discloses a "Method for purifying aromatic compounds nitrated by nitration process." However, there is no description made regarding the "composition for preventing, ameliorating, or treating a disease caused by protein nitration comprising a peptide with terminal tyrosine as effective component" as described in the present invention.

DETAILED DESCRIPTION OF THE INVENTION

TECHNICAL PROBLEMS TO BE SOLVED

The present invention has been devised under the aforementioned circumstances, and the inventors provide a composition for preventing, ameliorating, or treating a disease caused by protein nitration comprising a peptide with terminal tyrosine as effective component. By finding that the peptide with terminal tyrosine, which is the effective component of the present invention, inhibits protein nitration and also can prevent, ameliorate, or treat disease symptoms in chronic restraint stress-induced depression/cognitive impairment model, Alzheimer's disease model, epileptic seizure model, stroke model, type 2 diabetes model, acute renal failure model, or hyperammonemia model, the present invention is completed.

TECHNICAL MEANS FOR SOLVING THE PROBLEMS

To achieve the purpose described in the above, the present invention provides a functional health food composition for preventing or ameliorating a disease caused by protein nitration comprising a peptide with terminal tyrosine or a salt thereof, which is acceptable for use in food, as effective component.

The present invention further provides a pharmaceutical composition for preventing or treating a disease caused by protein nitration comprising a peptide with terminal tyrosine or a pharmaceutically acceptable salt thereof as effective component.

The present invention further provides a composition for inhibiting protein nitration comprising a peptide with terminal tyrosine or a pharmaceutically acceptable salt thereof as effective component.

The present invention further provides a method for removing a nitro group from nitrotyrosine by treating a protein having nitrotyrosine with a peptide with terminal tyrosine.

The present invention further provides a functional health food composition for preventing or ameliorating a disease caused by an increase in reactive oxygen species/reactive nitrogen species comprising a peptide with terminal tyrosine or a salt thereof, which is acceptable for use in food, as effective component.

The present invention still further provides a pharmaceutical composition for preventing or treating a disease caused by an increase in reactive oxygen species/reactive nitrogen species comprising a peptide with terminal tyrosine or a pharmaceutically acceptable salt thereof as effective component.

ADVANTAGEOUS EFFECT OF THE INVENTION

The peptide with terminal tyrosine, i.e., a peptide which has tyrosine on at least one peptide chain terminal, has an excellent effect of inhibiting the protein nitration, reducing the nitration level of tyrosine in glutamine synthetase that has been increased by stress, and increasing the activity of glutamine synthetase that has been decreased by stress, and also an excellent effect of preventing, ameliorating, or treating disease symptoms in chronic restraint stress-induced depression/cognitive impairment model, Alzheimer's disease model, epileptic seizure model, stroke model, type 2 diabetes model, acute renal failure model, or hyperammonemia model.

BRIEF DESCRIPTION OF THE DRAWINGS

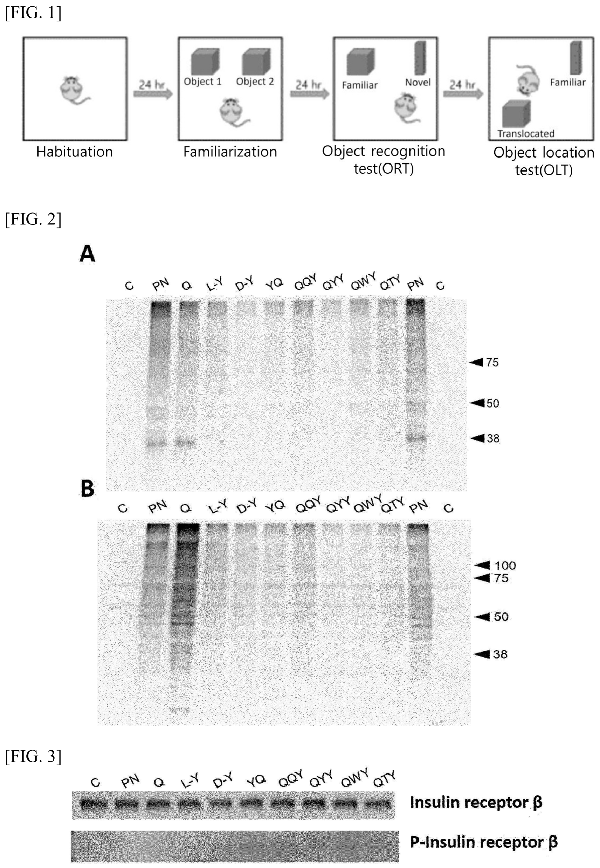

FIG. 1 illustrates the process of object recognition test (ORT) and object location recognition test (OLT) that are conducted to analyze the long-term memory ability of animals following the administration of peptide of the present invention.

FIG. 2 illustrates the result of determining the inhibiting effect of the peptide of the present invention on protein nitration. Panel A shows the Western blot results for nitrotyrosine protein in the prefrontal cortex (PFC) tissue, and Panel B shows the Western blot results for nitrotyrosine protein in the liver tissue.

FIG. 3 illustrates the Western blot results for insulin receptor beta and phosphorylated insulin receptor beta proteins in liver tissue for determining the regulation effect of the peptide of the present invention on protein expression level.

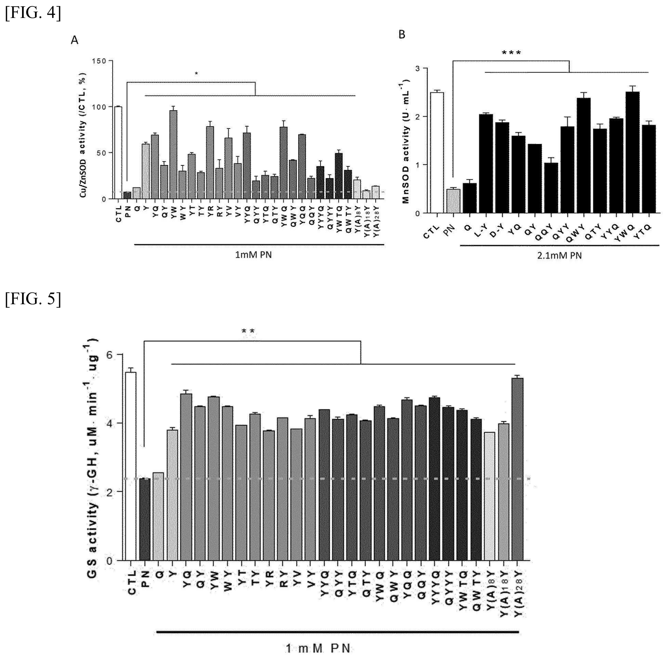

FIG. 4 illustrates the results of determining the inhibitory effect of the peptide with terminal tyrosine on the nitration of Cu/ZnSOD (A) and MnSOD (B). PN represents peroxynitrite, which induces protein nitration. * and *** indicate a statistically significant increase in the activity of Cu/ZnSOD or MnSOD in the peptide treatment group compared to the group treated only with PN, in which * represents $p < 0.05$ and *** represents $p < 0.001$.

FIG. 5 illustrates the results of determining the inhibitory effect of the peptide with terminal tyrosine on the nitration of glutamine synthetase. PN represents peroxynitrite, which induces protein nitration. ** indicates a statistically significant increase in the activity of glutamine synthetase in the peptide treatment group compared to the group treated only with PN, with $p < 0.01$.

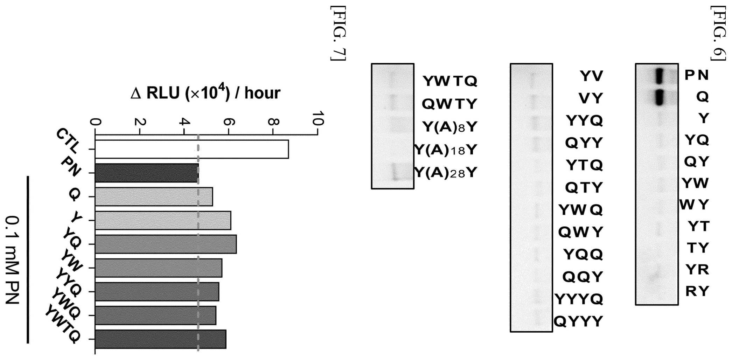

FIG. 6 illustrates the Western blot results for determining the inhibitory effect of the peptide with terminal tyrosine on the nitration of catalase.

FIG. 7 illustrates the results of determining the inhibitory effect of the peptide with terminal tyrosine on the nitration in which the determination was made by measuring the refolding activity of HSP60 (Heat Shock Protein 60). RLU represents the difference in absorbance values measured at Time 0 and Time 60 (relative light unit).

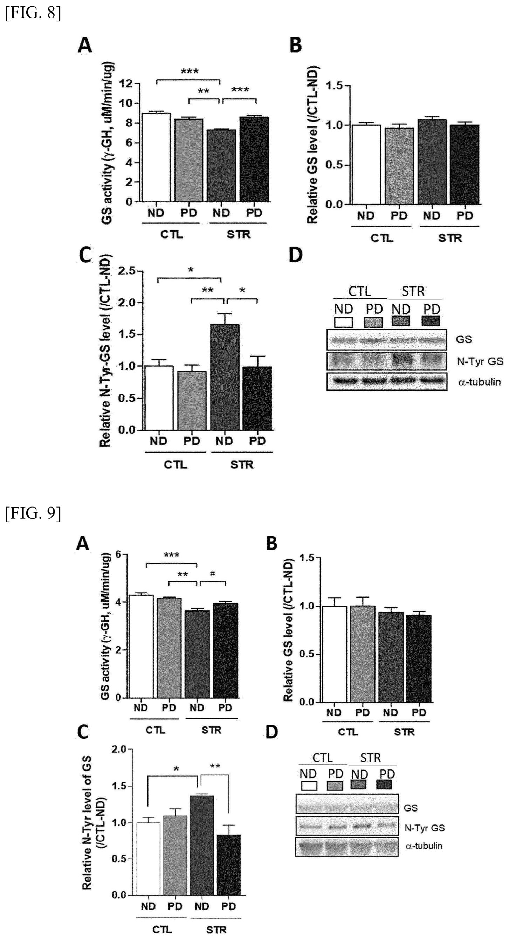

FIG. 8 illustrates the results of determining the effects of tyrosine-glutamine peptide diet (PD) in the chronic restraint stress treatment group (STR) on GS activity (A), GS expression level (B), and nitration level of tyrosine within GS (C and D).

FIG. 9 illustrates the results of determining the effects of glutamine-tyrosine peptide diet (PD) in the chronic restraint stress treatment group (STR) on GS activity (A), GS expression level (B), and nitration level of tyrosine within GS (C and D).

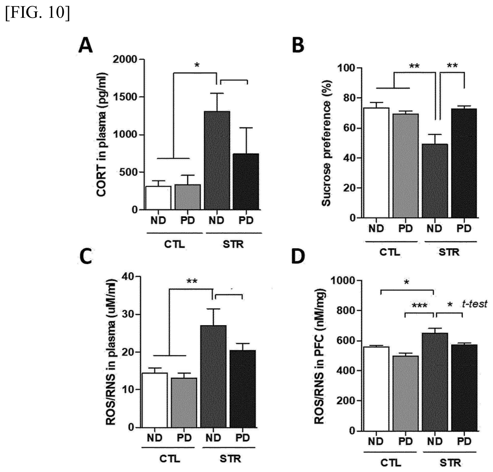

FIG. 10 illustrates the results of measuring the plasma corticosterone concentration (A), sucrose preference (B), plasma ROS/RNS concentration (C), and ROS/RNS concentration in the PFC tissues (D) in the chronic restraint stress treatment group (STR) following tyrosine-glutamine peptide diet (PD).

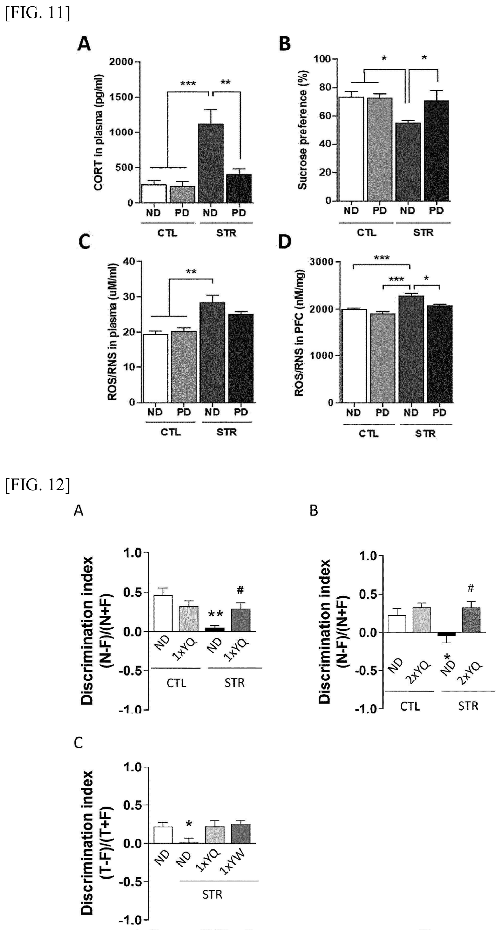

FIG. 11 illustrates the results of measuring the plasma corticosterone concentration (A), sucrose preference (B), plasma ROS/RNS concentration (C), and ROS/RNS concentration in the PFC tissues (D) in the chronic restraint stress treatment group (STR) following glutamine-tyrosine peptide diet (PD).

FIG. 12 illustrates the results of the analysis of memory performance in a chronic restraint stress animal model, following either a tyrosine-glutamine (YQ) peptide diet or a tyrosine-tryptophan (YW) peptide diet, in which A displays the results of the object recognition test (ORT) following 1xYQ peptide diet, B shows the ORT results following 2xYQ peptide diet, and C presents the results of the object location recognition test (OLT) following 1xYQ or 1xYW peptide diet, with A, B, and C as discrimination index. * and ** indicate a statistically significant decrease in the object recognition index or object location recognition index in the stress treatment group (STR) compared to the control group (CTL), in which * represents $p < 0.05$ and ** represents $p < 0.01$. # indicates that, in the stress treatment group, there is a statistically significant increase in the object recognition index in the peptide diet group (1xYQ or 2xYQ) compared to the normal diet group (ND), with $p < 0.05$.

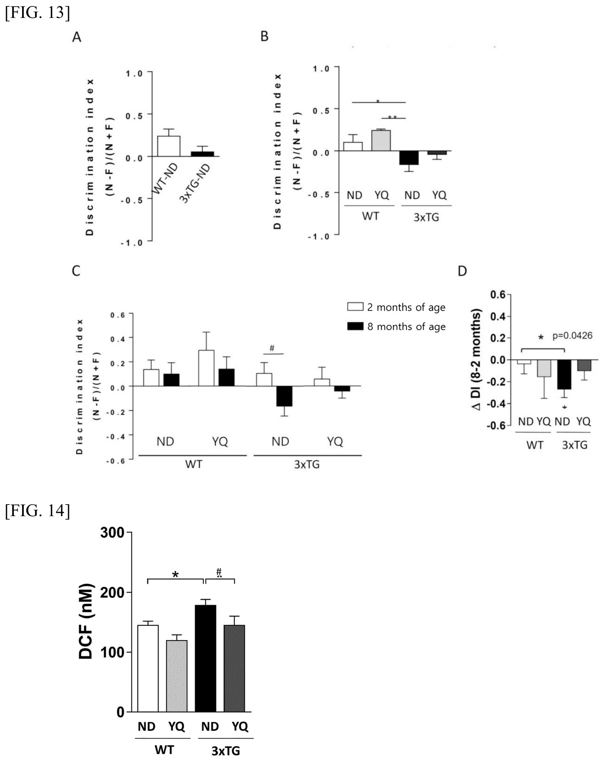

FIG. 13 illustrates the results of the object recognition test (ORT) conducted on an Alzheimer's disease animal model following a tyrosine-glutamine (YQ) peptide diet. Panel A shows the results of the test performed on animals at 2 months of age, panel B shows the results of the test on animals at 8 months of age, and panel C displays the results of the test conducted on animals at 2 months and also 8 months of age. Panel D provides the result about the magnitude of cognitive decline over a 6-month period. * and ** indicate a statistically significant decrease in the object recognition index in the Alzheimer's disease animal model (3xTG) compared to the control group (WT). Specifically, * represents $p<0.05$, and ** represents $p<0.01$. # indicates a statistically significant decrease in the object recognition index in animals of 8 months of age compared to animals at 2 months of age and, with $p<0.05$.

FIG. 14 illustrates the ROS/RNS concentration in an Alzheimer's disease animal model following a tyrosine-glutamine (YQ) peptide diet. * indicates a statistically significant increase in ROS/RNS concentration in the Alzheimer's disease model (3xTG) compared to the control group (WT), with $p<0.05$. # indicates that, in the Alzheimer's disease model, there is a statistically significant decrease in ROS/RNS concentration in the YQ peptide diet group compared to the normal diet group (ND), with $p<0.05$. DCF stands for 2',7'-dichlorofluorescin.

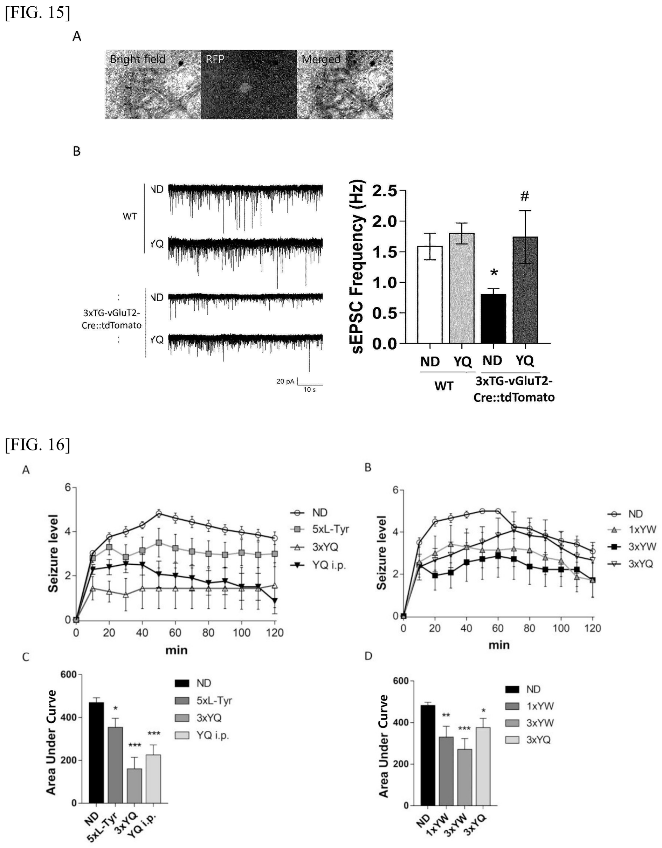

FIG. 15 illustrates the result obtained from fluorescent labeling of glutamatergic neuron in an Alzheimer's disease animal model. FIG. 15A illustrates the result showing that the animal model used is indeed a model of which glutamatergic neurons can be labeled in red. FIG. 15B shows the activity of glutamatergic neurons in accordance with the tyrosine-glutamine (YQ) peptide diet. * indicates a statistically significant decrease in the activity of glutamatergic neurons labeled with a fluorescent marker in the Alzheimer's disease animal model (3xTG-vGluT2-Cre::tdTomato) compared to the control group (WT), with $p<0.05$. # indicates that, in the Alzheimer's disease animal model, there is a statistically significant increase in the activity of glutamatergic neurons labeled with a fluorescent marker in the peptide diet group (YQ) compared to the normal diet group (ND), with $p<0.05$.

FIG. 16 illustrates the seizure level (A, B) and the area under the seizure level curve (C, D) in an animal model of epileptic seizure following a treatment with tyrosine-glutamine (YQ) or tyrosine-tryptophan (YW) peptide. *, **, and *** indicate that the seizure level in the peptide treatment groups (5xL-Tyr, 3xYQ, YQ i.p, 1xYW, or 3xYQ) was statistically significantly decreased compared to the normal diet group (ND), with statistical significance denoted by * ($p<0.05$), ** ($p<0.01$), and *** ($p<0.001$).

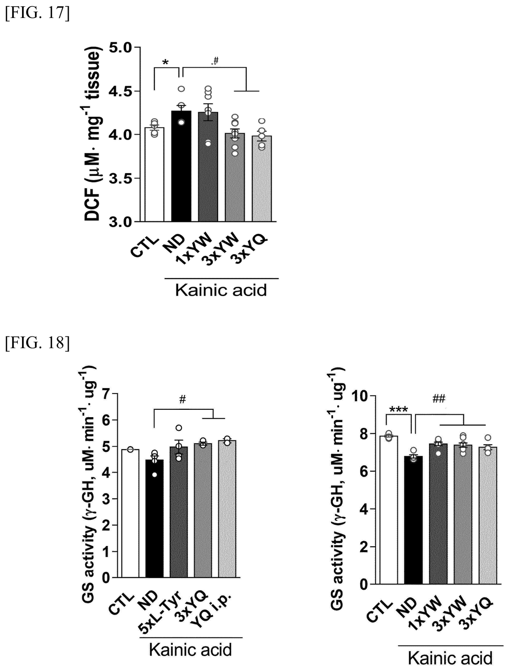

FIG. 17 illustrates the ROS/RNS concentration in an animal model of epileptic seizure following a treatment with tyrosine-glutamine (YQ) or tyrosine-tryptophan (YW) peptide. * indicates that the ROS/RNS concentration in the kainic acid treatment group was statistically significantly increased compared to the control group (CTL), with $p<0.05$. # indicates that, in the kainic acid-treated animals, the ROS/RNS concentration in the peptide treatment group (3xYW or 3xYQ) was statistically significantly decreased compared to the normal diet group (ND), with $p<0.05$.

FIG. 18 illustrates the activity of glutamine synthetase in an animal model of epileptic seizure following a treatment with tyrosine-glutamine (YQ) or tyrosine-tryptophan (YW) peptide. *** indicates that the activity of glutamine synthetase in the kainic acid treatment group was statistically significantly decreased compared to the control group (CTL), with $p<0.001$. # and ## indicate that, in the kainic acid-treated animals, the activity of glutamine synthetase in the peptide treatment group (3xYQ, YQ i.p, 1xYW, 3xYW, or 3xYQ) was statistically significantly increased compared to the normal diet group (ND), with $p<0.05$ for # and $p<0.01$ for ##.

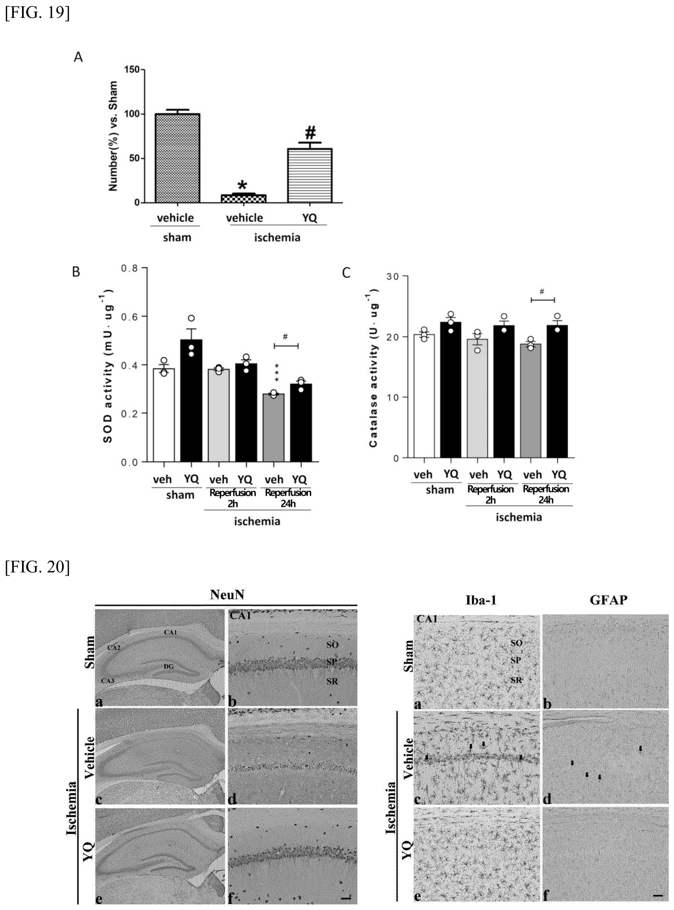

FIG. 19 illustrates the number of neurons (A), activity of superoxide dismutase (SOD) (B), and activity of catalase (C) in an animal model of ischemic stroke following a treatment with tyrosine-glutamine (YQ) peptide. Sham refers to the sham operation group, and vehicle refers to the group treated with 0.9% saline injection. * and *** indicate that the number of neurons, SOD activity, or catalase activity in the ischemic stroke animal model was statistically significantly decreased compared to the sham group, with $p<0.05$ for * and $p<0.001$ for ***. # indicates that, in the ischemic stroke animal model, the number of neurons, SOD activity, or catalase activity in the peptide treatment group (YQ) was statistically significantly increased compared to the vehicle group (injected with 0.9% saline), with $p<0.05$.

FIG. 20 illustrates the results of immunohistochemical staining for evaluating neuronal cell death in an animal model of ischemic stroke after a treatment with tyrosine-glutamine (YQ) peptide. Sham refers to the sham operation group, vehicle refers to the group treated with 0.9% saline injection, and ischemia refers to the animal model of ischemic stroke. NeuN represents the staining of pyramidal neuronal cells, Iba-1 represents of the staining of microglial cells, and GFAP represents the staining of astrocytes.

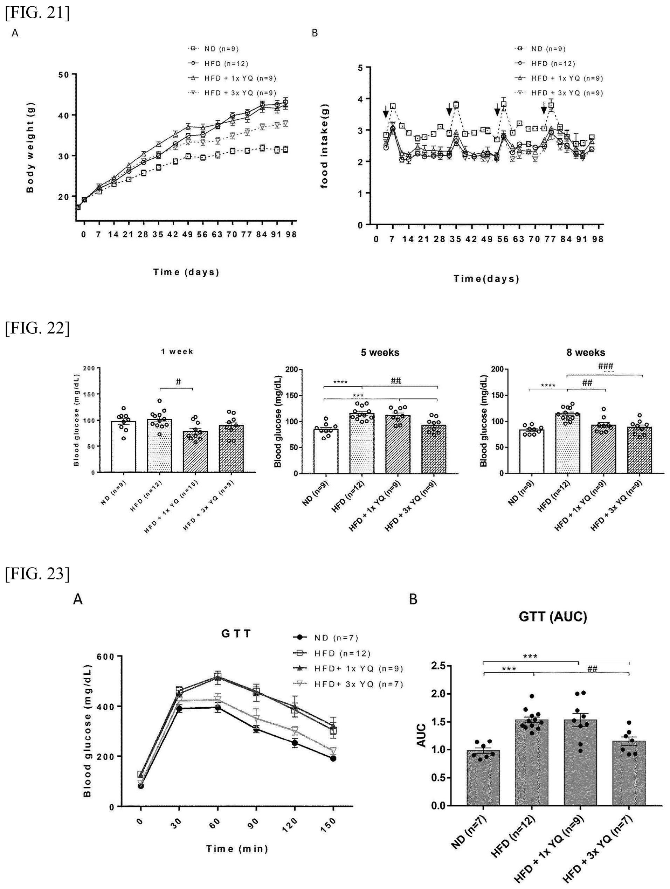

FIG. 21 displays the results of determining weight changes (A) and feed intake (B) over time in an animal model of type 2 diabetes caused by high-fat diet after a treatment with tyrosine-glutamine (YQ) peptide. ND refers to the normal diet group, and HFD refers to the high-fat diet group.

FIG. 22 illustrates the results of determining blood glucose level over time in an animal model of type 2 diabetes caused by high-fat diet after a treatment with tyrosine-glutamine (YQ) peptide. ND refers to the normal diet group, and HFD refers to the high-fat diet group. *** and **** indicate that blood glucose level was statistically significantly increased in the high-fat diet group (HFD) compared to the normal diet group (ND), with *** having $p<0.001$ and **** having $p<0.0001$. ## and ### indicate that blood glucose level was statistically significantly decreased in the high-fat diet group with YQ peptide supplementation (HFD+1xYQ or HFD+3xYQ) compared to the high-fat diet group (HFD), with ## having $p<0.01$ and ### having $p<0.001$.

FIG. 23 illustrates the results of a glucose tolerance test (GTT) in an animal model of type 2 diabetes caused by high-fat diet after a treatment with tyrosine-glutamine (YQ) peptide. FIG. 23A shows the blood glucose level over time, and B illustrates the area under the curve (AUC) in A. *** indicates that blood glucose level was statistically significantly increased in the high-fat diet group (HFD) compared to the normal diet group (ND), with *** having $p<0.001$. ## indicates that blood glucose level was statistically significantly decreased in the high-fat diet group with YQ peptide supplementation (HFD+3xYQ) compared to the high-fat diet group (HFD), with $p<0.01$.

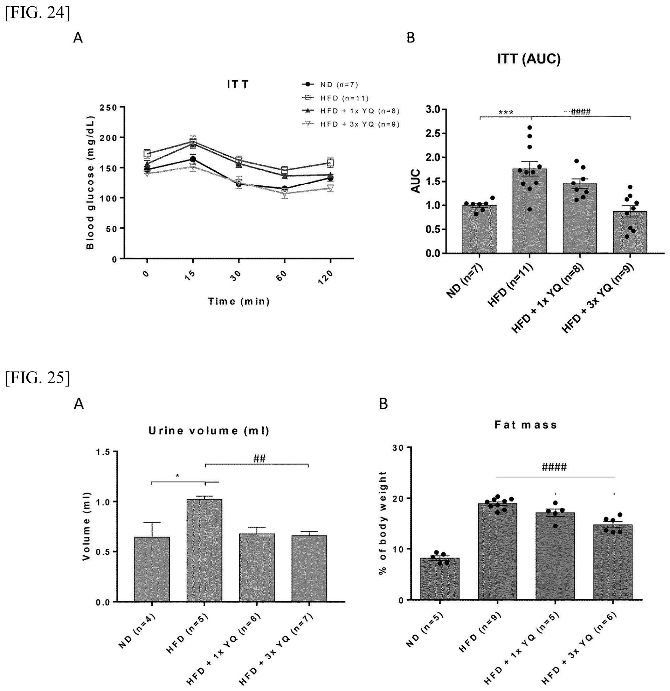

FIG. 24 illustrates the results of an insulin tolerance test (ITT) in an animal model of type 2 diabetes caused by high-fat diet after a treatment with tyrosine-glutamine (YQ) peptide. FIG. 24A shows the blood glucose level over time, and FIG. 24B illustrates the area under the curve (AUC) in A. *** indicates that blood glucose level was statistically significantly increased in the high-fat diet group (HFD) compared to the normal diet group (ND), with $p<0.001$. #### indicates that blood glucose level was statistically significantly decreased in the high-fat diet group with YQ peptide supplementation (HFD+3xYQ) compared to the high-fat diet group (HFD), with $p<0.0001$.

FIG. 25 illustrates the results of determining urine volume (A) and fat mass (B) in an animal model of type 2 diabetes caused by high-fat diet after a treatment with tyrosine-glutamine (YQ) peptide. * indicates that urine volume or fat mass was statistically significantly increased in the high-fat diet group (HFD) compared to the normal diet group (ND), with $p<0.05$. ## and #### indicate that urine volume or fat mass was statistically significantly decreased in the high-fat diet group with YQ peptide supplementation (HFD+3xYQ) compared to the high-fat diet group (HFD), with ## having $p<0.01$ and #### having $p<0.0001$.

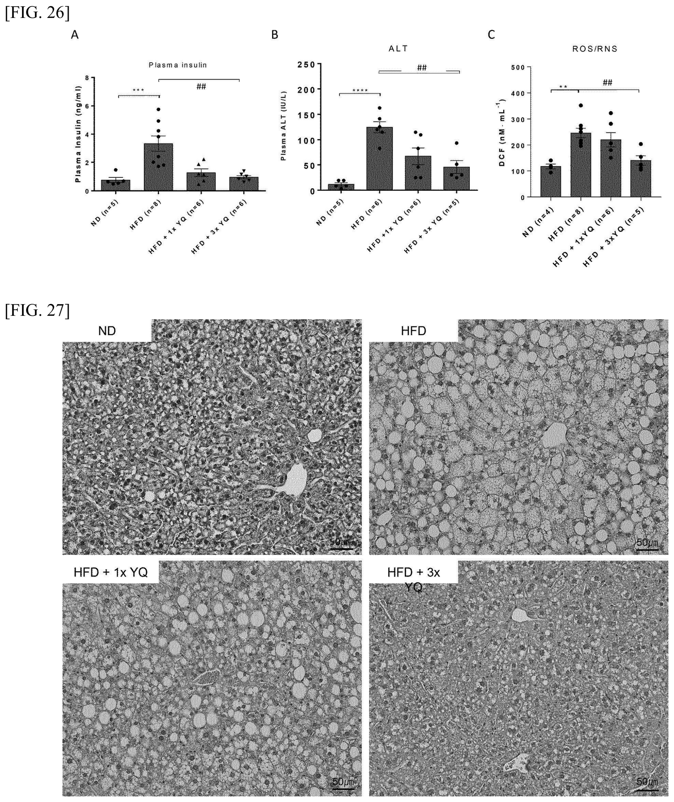

FIG. 26 illustrates the results of determining plasma insulin (A), ALT (B), and ROS/RNS (C) concentrations in an animal model of type 2 diabetes caused by high-fat diet after a treatment with tyrosine-glutamine (YQ) peptide. **, ***, and **** indicate that plasma insulin, ALT, or ROS/RNS concentration was statistically significantly increased in the high-fat diet group (HFD) compared to the normal diet group (ND), with ** having $p<0.01$, *** having $p<0.001$, and **** having $p<0.0001$. ## indicates that plasma insulin, ALT, or ROS/RNS concentration was statistically significantly decreased in the high-fat diet group with YQ peptide supplementation (HFD+3xYQ) compared to the high-fat diet group (HFD), with $p<0.01$.

FIG. 27 illustrates the H&E staining results of liver tissues for examining the changes in an animal model of type 2 diabetes caused by high-fat diet after a treatment with tyrosine-glutamine (YQ) peptide. ND refers to the normal diet group, while HFD refers to the high-fat diet group.

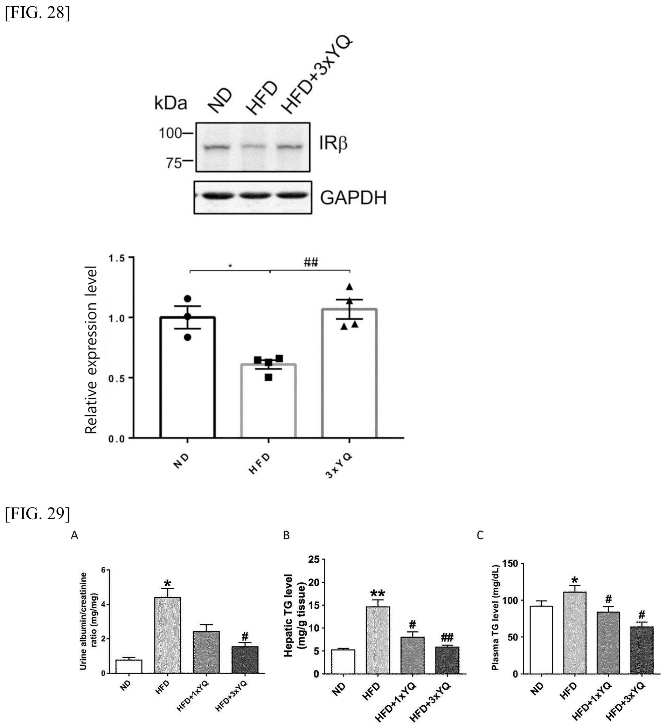

FIG. 28 illustrates the results of determining the expression level of insulin receptor beta (IRβ) in an animal model of type 2 diabetes caused by high-fat diet after a treatment with tyrosine-glutamine (YQ) peptide. * indicates a statistically significant decrease in IRβ expression level in the high-fat diet group (HFD) compared to the normal diet group (ND), with $p<0.05$. ## indicates a statistically significant increase in IRβ expression level in the high-fat diet group with YQ peptide supplementation (HFD+3xYQ) compared to the high-fat diet group (HFD), with $p<0.01$.

FIG. 29 illustrates the results of determining the ratio of urinary albumin/creatinine (A), the amount of triglycerides in liver tissues (B), and the amount of plasma triglycerides (C) in an animal model of type 2 diabetes caused by high-fat diet after a treatment with tyrosine-glutamine (YQ) peptide. * and ** indicate a statistically significant increase in the urinary albumin/creatinine ratio, the amount of triglycerides in liver tissues, or the amount of plasma triglycerides in the high-fat diet group (HFD) compared to the normal diet group (ND), with * having $p<0.05$ and ** having $p<0.01$. # and ## indicate a statistically significant decrease in the urinary albumin/creatinine ratio, the amount of triglycerides in liver tissues, or the amount of plasma triglycerides in the high-fat diet group with YQ peptide supplementation (HFD+1xYQ or HFD+3xYQ) compared to the high-fat diet group (HFD), with # having $p<0.05$ and ## having $p<0.01$.

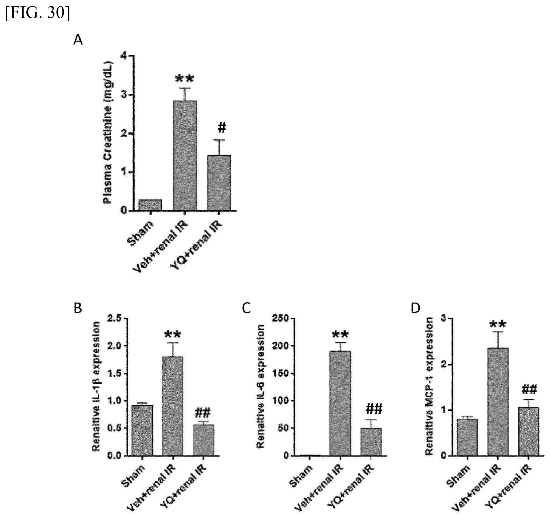

FIG. 30 illustrates the results of evaluating renal injury in an acute renal failure animal model after a treatment with tyrosine-glutamine (YQ) peptide. Specifically, the results include the concentration of creatinine in plasma (A) and the expression level of IL-1β (B), IL-6 (C), and MCP-1 (D) in renal tissues to evaluate the inflammatory response. Sham refers to the sham operation group, renal IR refers to the group of induced acute renal failure, and veh refers to the group receiving water administration. ** indicates a statistically significant increase in the concentration of creatinine, IL-1β, IL-6, or MCP-1 in the group of induced acute renal failure with water treatment (veh+renal IR) compared to the sham operation group (sham), with $p<0.01$. # and ## indicate a statistically significant decrease in the concentration of creatinine, IL-1β, IL-6, or MCP-1 in the group of induced acute renal failure with YQ peptide treatment (YQ+renal IR) compared to the group of induced acute renal failure with water treatment (veh+renal IR), with # having $p<0.05$ and ## having $p<0.01$.

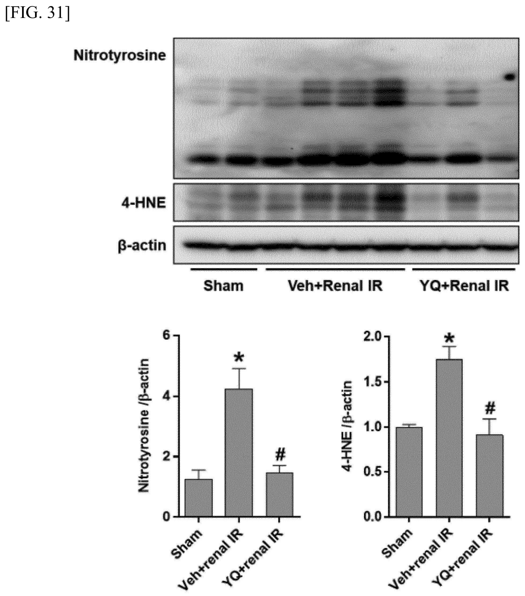

FIG. 31 illustrates the results of evaluating the expression level of nitrotyrosine and product of lipid peroxidation (i.e., 4-hydroxynonenal; 4-HNE) in renal tissues in an acute renal failure animal model after a treatment with tyrosine-glutamine (YQ) peptide. * indicates a statistically significant increase in the expression level of nitrotyrosine and product of lipid peroxidation (4-HNE) in the group of induced acute renal failure with water treatment (veh+renal IR) compared to the sham operation group (sham), with $p<0.05$. # indicates a statistically significant decrease in the expression level of nitrotyrosine and product of lipid peroxidation (4-HNE) in the group of induced acute renal failure with YQ peptide treatment (YQ+renal IR) compared to the group of induced acute renal failure with water treatment (veh+renal IR), with $p<0.05$.

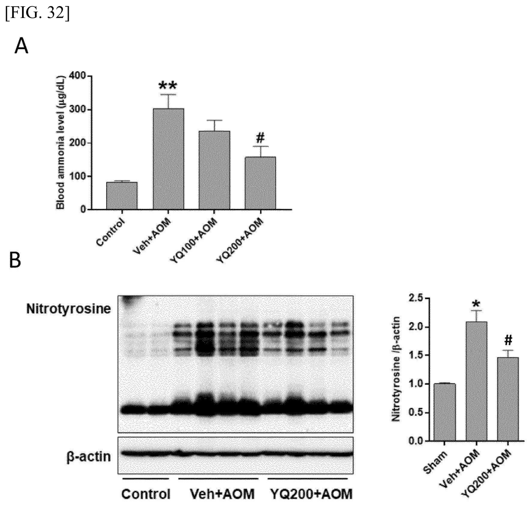

FIG. 32 illustrates the results of evaluating the serum ammonia concentration (A) and the expression level of nitrotyrosine in liver tissues in a hyperammonemia animal model after a treatment with tyrosine-glutamine (YQ) peptide. Control indicates the normal control group, and AOM indicates the hyperammonemia group induced by azoxymethane. * and ** indicate a statistically significant increase in the serum ammonia concentration and the expression level of nitrotyrosine in liver tissues in the group of induced hyperammonemia with water treatment (veh+AOM) compared to the normal control group (Control), with * having $p<0.05$ and ** having $p<0.01$. # indicates a statistically significant decrease in the serum ammonia concentration and the expression level of nitrotyrosine in liver tissues in the group of induced hyperammonemia with YQ peptide treatment (YQ200+AOM) compared to the group of induced hyperammonemia with water treatment (veh+AOM), with $p<0.05$.

## BEST MODE (S) FOR CARRYING OUT THE INVENTION

To achieve the purpose described in the above, the present invention provides a functional health food composition for preventing or ameliorating a disease caused by protein nitration comprising a peptide with terminal tyrosine or a salt thereof, which is acceptable for use in food, as effective component.

With regard to the peptide with terminal tyrosine, it can have tyrosine positioned at both terminals, or at one terminal. The amino acid linked to tyrosine can be polar/hydrophilic (e.g., tyrosine, glutamine, threonine), nonpolar/hydrophobic (e.g., tryptophan, valine), or charged (e.g., arginine). The number of connected amino acids can range from 1 to 29, but it is not limited thereto.

With regard to the functional health food composition for preventing or ameliorating a disease caused by protein nitration, it is preferable that the protein is any one selected from glutamine synthetase, insulin receptor $\beta$ subunit, Mn superoxide dismutase, heat shock protein 60, Cu/Zn superoxide dismutase, and catalase, but it is not limited thereto.

Furthermore, examples of the disease caused by the protein nitration include depressive disorder, anxiety disorder, stroke, epilepsy, seizure, cognitive impairment, Alzheimer's disease, dementia, type 2 diabetes, diabetic nephropathy, muscular dystrophy, dyslipidemia, obesity, non-alcoholic fatty liver disease, acute kidney injury, hyperammonemia, and hepatic encephalopathy, but it is not limited thereto.

The functional health food composition for preventing or ameliorating a disease caused by protein nitration can be prepared in any formulation selected from a pill, a tablet, a capsule, a powder preparation, a powder, a granule, a candy, a syrup, and a drink, but it is not limited thereto. Alternatively, the composition can be added as a food component, and it can be suitably prepared by following a common method.

Examples of the food product to which the effective component of the present invention can be added include meat, sausage, bread, chocolate, candies, snacks, biscuits, pizza, ramen, other noodles, gums, dairy products including ice cream, various kinds of soup, beverage, tea, drink, alcohol beverage, and vitamin complex, and all functional health food products in general sense are included therein.

The functional health food composition may further comprise various nutritional supplements, a vitamin, a mineral (i.e., electrolyte), a natural or synthetic flavor, a coloring agent, an enhancing agent (e.g., cheese and chocolate), pectinic acid and a salt thereof, alginic acid and a salt thereof, an organic acid, a protective colloidal thickening agent, a pH adjusting agent, a stabilizer, a preservative, glycerin, alcohol, and a carbonating agent used for carbonated drink. Other than those, fruit juice or fruit pulp for producing vegetable drink may be additionally comprised. Those ingredients may be used either singly or in combination thereof.

Furthermore, the functional health food composition of the present invention may comprise various flavoring agents and natural carbohydrates as an additional component. Examples of the natural carbohydrates include monosaccharides like glucose and fructose, disaccharides like maltose and sucrose, polysaccharides like dextrin and cyclodextrin, and sugar alcohols like xylitol, sorbitol, and erythritol. As a sweetening agent, a natural sweetening agent like taumatin and stevia extract and a synthetic sweetening agent like saccharine and aspartame can be used.

The present invention further provides a pharmaceutical composition for preventing or treating a disease caused by protein nitration comprising a peptide with terminal tyrosine or a pharmaceutically acceptable salt thereof as effective component.

With regard to the pharmaceutical composition for preventing or treating a disease caused by protein nitration, the protein and disease caused by protein nitration are the same as those described in the above.

In addition to the protein with terminal tyrosine, a pharmaceutically acceptable carrier, vehicle, or diluent may be further comprised in the pharmaceutical composition. The pharmaceutical composition of the present invention can be administered either orally or parenterally, and when administered parenterally, it can be applied by topical application on skin or an intraperitoneal, rectal, intravenous, intramuscular, or subcutaneous injection method can be selected, but it is not limited thereto.

The pharmaceutical composition of the present invention can be formulated using diluents or excipients such as fillers, bulking agents, binding agents, wetting agents, disintegrants, surfactants and the like. Solid preparations for oral administration may include tablets, pills, powders, granules, capsules, and the like. These solid preparations are formulated by mixing one or more compounds with at least one excipient, such as starch, calcium carbonate, sucrose, lactose, or gelatin. In addition to simple excipients, lubricants such as magnesium stearate and talc may also be used. Liquid preparations for oral administration include suspensions, solutions, emulsions, syrups, and the like. In addition to commonly used diluents such as water and liquid paraffin, various excipients such as wetting agents, sweeteners, flavoring agents, and preservatives may be included. Preparations for parenteral administration may include sterile solutions, non-aqueous vehicles, suspensions, emulsions, freeze-dried formulations, and suppositories. As non-aqueous solvents and suspending agents, propylene glycol, polyethylene glycol, vegetable oils (e.g., olive oil), injectable esters (e.g., ethyl oleate) and the like can be used. As suppository bases, Witepsol, Macrogol, Tween 61, cocoa butter, laurin gelatin, and glycerol and the like can be used.

The pharmaceutical composition according to the present invention is administered in a pharmacologically effective amount. In the present invention, "pharmacologically effective amount" refers to an amount sufficient to treat a disease with a reasonable benefit/risk ratio applicable to medical therapy. The effective dosage level can be determined based on various factors, including the type and severity of the patient's condition, the activity of the drug, sensitivity to the drug, administration time, route of administration, elimination rate, treatment duration, factors including concomitant use of other drugs, and other well-known factors in the medical field. The pharmaceutical composition of the present invention can be administered as an individual therapeutic agent or in combination with other therapeutic agents, sequentially or simultaneously with conventional therapies, and in a single or multiple doses. It is important to administer the minimum effective amount that can achieve maximum efficacy without adverse effects, and this can be readily determined by a person who is skilled in the pertinent art.

The dosage of the composition of the present invention can vary in range depending on the patient's weight, age, gender, health condition, diet, administration time, method of administration, elimination rate, and severity of the disease.

The present invention further provides a composition for inhibiting tyrosine nitration in protein comprising a peptide with terminal tyrosine or a pharmaceutically acceptable salt thereof as effective component.

With regard to the composition for inhibiting tyrosine nitration in protein, the protein is the same as those described in the above.

The present invention further provides a method for removing a nitro group from nitrotyrosine by treating a protein having nitrotyrosine with a peptide with terminal tyrosine.

The present invention further provides a functional health food composition for preventing or ameliorating a disease caused by an increase in reactive oxygen species/reactive nitrogen species comprising a peptide with terminal tyrosine or a salt thereof, which is acceptable for use in food, as effective component.

The present invention still further provides a pharmaceutical composition for preventing or treating a disease caused by an increase in reactive oxygen species/reactive nitrogen species comprising a peptide with terminal tyrosine or a pharmaceutically acceptable salt thereof as effective component.

With regard to the pharmaceutical composition for preventing or treating a disease caused by an increase in reactive oxygen species/reactive nitrogen species, the disease caused by an increase in reactive oxygen species/reactive nitrogen species is preferably any one selected from depressive disorder, anxiety disorder, stroke, epilepsy, seizure, cognitive impairment, Alzheimer's disease, dementia, type 2 diabetes, diabetic nephropathy, muscular atrophy, dyslipidemia, obesity, non-alcoholic fatty liver disease, acute kidney injury, hyperammonemia, and hepatic encephalopathy, but it is not limited thereto.

Hereinbelow, the present invention is explained in greater detail in view of the Examples. However, the following Examples are given only for specific explanation of the present invention and it would be evident to a person who has common knowledge in the pertinent art that the scope of the present invention is not limited by them.

EXAMPLES

Materials and Methods

1. Peptide synthesis

To analyze the effects of the peptide according to the present invention, glutamine (Q), L-tyrosine (L-Y), or D-tyrosine (D-Y) were used as individual amino acid. As for the peptide with terminal tyrosine, the followings were used: tyrosine-glutamine (YQ), glutamine-tyrosine (QY), tyrosine-tryptophan (YW), tryptophan-tyrosine (WY), tyrosine-threonine (YT), threonine-tyrosine (TY), tyrosine-arginine (YR), arginine-tyrosine (RY), tyrosine-valine (YV), valine-tyrosine (VY), tyrosine-tyrosine-glutamine (YYQ), glutamine-tyrosine-tyrosine (QYY), tyrosine-threonine-glutamine (YTQ), glutamine-threonine-tyrosine (QTY), tyrosine-tryptophan-glutamine (YWQ), glutamine-tryptophan-tyrosine (QWY), tyrosine-glutamine-glutamine (YQQ), glutamine-glutamine-tyrosine (QQY), tyrosine-tyrosine-tyrosine-glutamine (YYYQ), glutamine-tyrosine-tyrosine-tyrosine (QYYY), tyrosine-tryptophan-threonine-glutamine (YWTQ), glutamine-tryptophan-threonine-tyrosine (QWTY), tyrosine-8 random amino acids-tyrosine ($Y(A)_8Y$), tyrosine-18 random amino acids-tyrosine ($Y(A)_{18}Y$), or tyrosine-28 random amino acids-tyrosine ($Y(A)_{28}Y$). As for the glutamine, glutamine from Nutricost (purity 100%) was used. As for the L-tyrosine, L-tyrosine from Sigma-Aldrich (#93829, >99.0% BioUltra) was used. As for the D-tyrosine, D-tyrosine from Sigma-Aldrich (#855456, >99.0% BioUltra) was used. The peptides used for *in vitro* experiments were synthesized by Peptron (purity >98%), and the peptides used for *in vivo* experiments were synthesized by GL Biochem (purity >95%).

2. Animal test

In the present invention, 7-week-old male C57BL/6 mice, 3xTG-AD mice (i.e., Alzheimer's disease model), 4-week-old male ICR mice, or Mongolian gerbils (65 g to 75 g) were kept at a temperature of 22-24°C, humidity of 50-70%, and a 12-hour light-dark cycle. The animals were provided ad libitum access to diet feed and water. The animals used in the present invention were handled in accordance with the protocol approved by the Gyeongsang National University Institutional Animal Care and Use Committee (GNU IACUC) under protocol number GNU-161128-M0068, which follows the guidelines of the National Institutes of Health (NIH, Bethesda, MD, USA).

3. Preparation of mouse brain and liver tissue samples

From the mice anesthetized with $CO_2$ gas, prefrontal cortex (PFC) and liver tissues were separately collected. The tissues were weighed, then homogenized using a tissue homogenizer. The homogenized tissues were centrifuged at 12,000 rpm for 20 minutes at 4°C, and the supernatant was collected to give lysates of PFC and liver tissues.

4. Preparation of feed supplemented with tyrosine-containing peptide

For carrying out the animal test following the peptide diet of the present invention, mouse feed supplemented with tyrosine-containing peptide was prepared. As for the tyrosine-containing peptide, tyrosine-glutamine (YQ), glutamine-tyrosine (QY), or tyrosine-tryptophan (YW), with molecular weights of 309.32 g/mol or 367.40 g/mol, was used. These peptides were added to a standard feed (AIN-93G) to prepare the feed (Table 1).

[Table 1]

| Feed type | Composition |
|---|---|
| 1xYQ or 1xQY | 309.32 mg YQ or QY + 1 kg standard feed |
| 2xYQ | 618.64 mg YQ + 1 kg standard feed |
| 3xYQ | 927.96 mg YQ + 1 kg standard feed |
| 1xYW | 367.40 mg YW + 1 kg standard feed |
| 3xYW | 1120.2 mg YW + 1 kg standard feed |

5. Cognitive function test

To analyze the long-term memory capacity of animals following the peptide diet of the present invention, both the object recognition test (ORT) and object location recognition test (OLT) were conducted (FIG. 1). The tests consisted of three phases: habituation, familiarization, and test. During the habituation phase, the animals were acclimated to the testing apparatus for 10 minutes per day over two days. On the third day at the familiarization phase, the animals were allowed to explore two identical objects for 10 minutes. Subsequently, on the fourth day at the test phase, one of the objects was replaced with a novel object, and the animals were allowed to explore for 10 minutes, with result recorded during the initial 5 minutes was used as test data. The object recognition test (ORT) index was calculated using the following formula.

$$\text{Discrimination index} = (N-F)/(N+F)$$

N: Time spent for exploring new object
F: Time spent for exploring familiar object

After 24 hours of performing the ORT, the location of the familiar object was changed, and the animals were allowed to explore for 5 minutes. During the total exploration time, the ratio of time spent exploring the object with a changed location was calculated to determine the object location recognition test (OLT) index.

Example 1. Analysis of inhibitory effect on protein nitration

1-1. Analysis of inhibitory effect on protein nitration in brain and liver tissues of mice

To analyze the inhibitory effect of the peptide of the present invention on protein nitration, lysates of mouse brain and liver tissues were used. Peroxynitrite (PN), which induces protein nitration, was added to the tissue lysates, and each of the peptides according to the present invention was added at a concentration of 2 mM. The mixture was vortexed for 5 seconds and then reacted on ice for 10 minutes. Subsequently, Western blot was performed using anti-nitrotyrosine antibody (1 : 1000), anti-insulin receptor beta antibody (1 : 1000), or anti-phospho insulin receptor beta antibody (1 : 1000).

The results showed an increase in protein with higher tyrosine nitration in the prefrontal cortex (PFC) and liver tissues as a result of a treatment with PN. This increase was identified by multiple protein bands in Western blot, showing increased nitration of various proteins other than glutamine synthetase (GS) and insulin receptor beta subunit (IRβ). It was also found that, in the proteins with tyrosine nitration increased by PN, the nitration could be reduced by a treatment with the peptide having terminal tyrosine (Y) (FIG. 2). Furthermore, in the liver tissues, there was no difference in the expression of IRβ protein between the presence and absence of a treatment with PN or the peptide containing terminal tyrosine (Y). On the other hand, it was also found that phospho-IRβ protein showed decreased expression due to nitration by PN, while its expression was increased by a treatment with the peptide having terminal tyrosine (Y) (FIG. 3).

1-2. Analysis of inhibitory effect on nitration of Cu/ZnSOD and MnSOD

To analyze the inhibitory effect of peptide according to the present invention on the nitration of Cu/Zn superoxide dismutase (SOD-1) and Mn superoxide dismutase (SOD-2), the SOD colorimetric assay kit (Thermo Scientific, #EIASODC) was employed. Recombinant human Cu/ZnSOD or MnSOD within the standard range was placed in a PCR tube, and the peptide according to the present invention was added at a concentration of 1 mM. Then, 1 mM of peroxynitrite (PN) was added, followed by mixing and incubating on ice for 10 minutes. Subsequently, 25 $\mu$L of xanthine oxidase provided in the kit were added, and the mixture was incubated at room temperature for 20 minutes. The absorbance was then measured at 450 nm. By subtracting the absorbance measured in the first measurement from the absorbance measured in the second measurement, the activity of Cu/ZnSOD or MnSOD was determined.

The results showed that the activity of Cu/ZnSOD or MnSOD significantly decreased upon PN treatment, but the activity of Cu/ZnSOD or MnSOD was restored when the protein was treated with the peptide with terminal tyrosine (FIG. 4).

1-3. Analysis of inhibitory effect on nitration of glutamine synthetase

To analyze the inhibitory effect of the peptide according to the present invention on the nitration of glutamine synthetase (GS), lysate of mouse brain tissue was used. The lysate of the PFC tissue was placed in a PCR tube, and the peptide according to the present invention was added at a concentration of 1 mM. Then, 1 mM of peroxynitrite (PN) was added, followed by mixing and incubating on ice for 10 minutes. Subsequently, 50 $\mu$L of the reacted sample were transferred to a 96-well plate, and 50 $\mu$L of the glutamine synthetase assay buffer (50 mM imidazole-HCl, pH 6.8, 25 mM L-glutamine, 12.5 mM hydroxylamine, 12.5 mM sodium arsenate, 1 mM MnCl$_2$, and 0.08 mM ADP) were added. The reaction was allowed to occur at 37°C for about 40 minutes, and the absorbance was measured at 560 nm. The activity of glutamine synthetase was then calculated using a standard curve generated with $\gamma$-glutamylhydroxamate.

The results showed that the activity of glutamine synthetase was significantly reduced by PN treatment. However, it was observed that the activity of glutamine synthetase was restored by the peptide with terminal tyrosine. Particularly, even from a long peptide consisting of 30 amino acids, the effect of restoring the activity of glutamine synthetase, which had been reduced by nitration caused by PN treatment, was exhibited (FIG. 5).

1-4. Analysis of inhibitory effect on nitration of catalase

To analyze the nitration inhibitory effect of the peptide according to the present invention on catalase, a Western blot using human recombinant catalase was performed. In PCR tube, 0.2 $\mu$g of human recombinant catalase was added, and it was further added with each peptide according to the present invention at a concentration of 2 mM. Then, 1 mM peroxynitrite (PN) was added, and the mixture was incubated on ice for 10 minutes. Subsequently, SDS sample buffer was added, and the sample was boiled for 5 minutes. The mixture was subjected to SDS-PAGE, followed by transfer onto a PVDF membrane. Western blot was then performed using an anti-nitrotyrosine antibody (1 : 1000).

The results showed that the expression of nitrated catalase increased upon PN treatment. However, it was observed that the expression of nitrated catalase decreased when it is treated with the peptide containing terminal tyrosine (FIG. 6).

1-5. Analysis of inhibitory effect on nitration based on measurement of refolding activity of HSP60 (heat shock protein 60)

Activity of HSP60, which is one kind of chaperonin proteins, is known to be inhibited by protein nitration, and several diseases associated with this phenomenon have been reported. In order to analyze the inhibitory effect of the peptide according to the present invention on the nitration of HSP60, the HSP60/HSP10 Glow-Fold Protein Refolding Kit (R&D Systems, #K-300) was utilized. In PCR tube, the HSP60 solution provided from the kit was mixed with the peptide according to the present invention at a concentration of 1 mM each. Then, 1 mM peroxynitrite (PN) was added, and the mixture was incubated on ice for 10 minutes. Subsequently, the HSP10 solution, $Mg^{2+}$-ATP solution, and Glow-Fold substrate protein, all provided in the kit, were added, and the reaction was allowed to occur at room temperature for 15 to 30 minutes. After the reaction, the sample was heated at 45°C for 7 minutes and immediately placed on ice. In a 96-well plate, a luciferin solution (50 $\mu$L) was mixed with the sample (4 $\mu$L), and luminescence was measured within 1 to 2 minutes using a microplate reader (Time 0). The remaining sample was further incubated at 30°C for 60 minutes, and then the luciferin solution (50 $\mu$L) was mixed with the sample (4 $\mu$L), followed by luminescence measurement within 1 to 2 minutes using a microplate reader (Time 60). The difference in absorbance values (RLU, relative light units) between Time 0 and Time 60 was used to analyze the refolding activity of HSP60 in each sample.

The results showed that the refolding activity of HSP60 was significantly decreased by PN treatment; however, it was restored by the peptide containing terminal tyrosine (FIG. 7). Based on these results, it is believed that, by using the peptide with terminal tyrosine, diseases induced by the reduced activity of HSP60 can be either prevented or ameliorated.

Example 2. Analysis of inhibitory effect on GS nitration and anti-depressant effect in animal model of chronic immobilization stress

A total of 28 male C57BL/6 mice at 7 weeks of age were divided into two groups: a control group (CTL) and a stress group (STR). The stress group was subjected to chronic immobilization stress for 15 days. Specifically, each mouse in the stress group was individually placed in a restrainer for 2 hours daily from 2 pm to 4 pm to induce chronic immobilization stress. The control group (CTL) and stress group (STR) were further classified based on their diets. Specifically, the mice in each group were either fed a normal diet (ND) with balanced nutrition or a peptide diet (PD) supplemented with YQ or QY peptide (330 mg/kg). After the completion of the 15-day chronic immobilization stress, the mice were subjected to a sucrose preference test (SPT). Blood samples and prefrontal cortex (PFC) tissues were collected from the mice for subsequent test.

(1) Analysis of inhibitory effect on GS nitration

Using the prefrontal cortex (PFC) lysate samples from mice obtained after the completion of chronic immobilization stress, GS activity and GS expression level were measured by following the method described above.

The results showed that GS expression level was similar, but GS activity was reduced by the stress. Additionally, among the stress groups, the peptide diet group supplemented with YQ or QY peptide showed a statistically significant increase in GS activity compared to the normal diet group (FIGs. 8A, 8B, 9A, and 9B).

Furthermore, the level of tyrosine nitration within GS was measured by using immunoprecipitation (IP)-WB. The immunoprecipitation analysis of tyrosine-nitrated GS was performed using an anti-nitrotyrosine antibody (ab61392, Abcam) and Protein A/G PLUS-Agarose (Santa Cruz) according to the manufacturer's protocol.

The results showed a statistically significant increase in the level of tyrosine nitration of GS induced by the stress. Moreover, among the stress groups, the peptide diet group supplemented with YQ or QY peptide exhibited a statistically significant decrease in tyrosine nitration level of GS compared to the normal diet group (FIGs. 8C, 8D, 9C, 9D).

(2) Analysis of anti-depressive effect

For the analysis of anti-depressive effect, blood and PFC tissues were collected from mice that underwent chronic immobilization stress. The blood collected from mice was centrifuged at 1,000 × g for 10 minutes at 4°C to separate the plasma, and the supernatant was collected and diluted 1/20 with PBS to prepare a sample. The corticosterone concentration in the plasma was then measured using the Corticosterone EIA kit (Cayman) by following the manufacturer's instructions.

The results showed that the corticosterone concentration in the plasma significantly increased due to the stress, and, among the stress groups, it was significantly decreased in the plasma of mice from the YQ or QY peptide diet group compared to the normal diet group (FIGs. 10A and 11A).

In addition, the plasma diluted with PBS at a 1/3 ratio and the PFC tissue lysate (50 $\mu$g) were prepared. The ROS/RNS (reactive oxygen species/reactive nitrogen species) level in the plasma and PFC tissues was measured using the ROS/RNS assay kit (Cell Biolabs) according to the manufacturer's instructions.

The results showed that the ROS/RNS level in both the plasma and PFC tissue significantly increased due to the stress, and it was also found that, among the stress groups, the ROS/RNS level decreased in the plasma and PFC tissue of mice from the YQ or QY peptide diet group compared to the normal diet group under stress conditions (FIGs. 10C, 10D, 11C, and 11D).

In addition, the depression was evaluated by measuring the sucrose preference of mice that had undergone 15 days of chronic immobilization stress. The sucrose preference test was conducted over a total of 4 days. Equal amounts of 0.1 M sucrose solution and water were provided in identical bottles of the same size and shape. On the first day, mice were given access to 0.1 M sucrose solution and water for 24 hours. On the second day, the positions of the sucrose solution and water bottles were switched, and mice were given access for 24 hours. On the third day, no sucrose solution or water was provided. On the fourth day, mice were again given access to equal amounts of 0.1 M sucrose solution and water for 3 hours, the positions were then switched, and access was provided for another 3 hours. The amount of sucrose solution and water consumed during the 6-hour period on the fourth day was measured, and the sucrose preference (%) was calculated according to the following formula.

Sucrose preference (%) = Sucrose intake amount/(Water intake amount + Sucrose intake amount)×100

The results showed a decrease in sucrose preference due to the stress, and it was observed that, among the stress groups, the sucrose preference was increased in the peptide diet group supplemented with YQ or QY peptide compared to the normal diet group (FIGs. 10B and 11B).

Example 3. Analysis of improvement in cognitive function in animal model of chronic immobilization stress

A total of 28 seven-week-old male C57BL/6 mice were divided into two groups: a control group and a stress group. The mice were provided with feed having tyrosine-containing peptide (1xYQ, 2xYQ, or 1xYW) for one week. Each animal of the stress group was separately forced to a restrainer to experience chronic stress, for 2 hours each day for two weeks (from 2 PM to 4 PM). After the stress period, an object recognition test (ORT) was performed to evaluate cognitive function.

The results showed a decrease in cognitive function due to the stress, and it was observed that, among the stress groups, the group fed with YQ or YW peptide-supplemented diet exhibited enhanced cognitive function compared to the normal diet group (FIG. 12).

Example 4. Analysis of improvement in cognitive function in animal model of Alzheimer's disease

4-1. Mouse model with modified Alzheimer's disease gene

The cognitive function of a 2-month-old mouse 3xTG-AD, which is a mouse model of modified Alzheimer's disease gene, and a 2-month-old normal C57BL/6 mouse was examined. After the examination, both were divided into two groups, and they were fed with either a normal diet (ND) or a diet supplemented with tyrosine-containing peptide (1xYQ). Additionally, at 8 months of age, known as the onset time point of mild cognitive impairment in 3xTG-AD mice, the cognitive function was examined again to evaluate the effect of tyrosine peptide on protecting cognitive function.

The results showed that the cognitive function in the animal model of dementia was decreased compared to the normal mice, and the cognitive function decline was more significant at 8 months of age compared to 2 months of age. Additionally, in the normal mouse group, there was no significant difference caused by the peptide diet. However, in the dementia animal model, it was found that the YQ peptide diet caused a slight improvement in cognitive function (FIG. 13).

Furthermore, 100 $\mu$l of RIPA buffer, containing proteinase and phosphatase inhibitors, per 10 mg of tissue weight was added on dissected hippocampal tissue. The RIPA tissue mix was homogenized using glass beads and a blender for 1 minute. The homogenate was then centrifuged at 12,000 $\times$ g, 4°C for 15 minutes, and the supernatant was collected. It was further diluted 10 times in PBS and used for the measurement of reactive oxygen species/reactive nitrogen species (ROS/RNS). The ROS/RNS level was measured using the OxiSelect ROS/RNS assay kit (Cell Biolabs) by following the recommended experimental protocol.

The results showed an increase in ROS/RNS level in the hippocampal tissues of the dementia animal model compared to the normal group, but the YQ peptide led to a decrease in the ROS/RNS level (FIG. 14).

4-2. Alzheimer's disease animal model with fluorescent labeled glutamatergic neuron

vGluT2-IRES-Cre::tdTomato mouse, which has fluorescent labeled glutamatergic neurons, was cross-bred with the 3xTG-AD mouse, which is an animal model with modified Alzheimer's disease gene, to yield 3xTG-vGluT2-Cre::tdTomato, which is a dementia model with fluorescent labeled glutamatergic neuron. Subsequently, it was identified as an animal model of which glutamatergic neuron can be labeled in red color (FIG. 15A).

At two months of age, 3xTG-vGluT2-Cre::tdTomato mice were fed with diet supplemented with tyrosine-containing peptide (1xYQ). At 6 to 8 months of age, the spontaneous excitatory postsynaptic current (sEPSC) was measured to evaluate the activity of glutamatergic neurons. For recording membrane currents, a 200 $\mu$m-thick brain cross-section was placed in a recording chamber filled with artificial cerebrospinal fluid at a rate of 1.5 to 2 ml/min, and, under a holding potential of -70 mV, whole-cell voltage clamp recordings were obtained from visually identified glutamatergic neurons in the medial prefrontal cortex (mPFC). Glutamatergic currents were isolated by the addition of picotroxin (100 $\mu$M). All recordings were performed at $30 \pm 2°C$, and, as the pipette solution, a solution containing 30 mM KCl, 5 mM CaCl$_2$, 10 mM EGTA, 10 mM HEPES, 2 mM MgATP, 0.5 mM Na$_2$GTP, and 5 mM phosphocreatine was used.

The results showed that the glutamatergic neuronal activity in the dementia animal model was decreased compared to the normal group. However, it was observed that the administration of YQ peptide increased the glutamatergic neuronal activity (FIG. 15B).

Example 5. Analysis of inhibitory effect on excitotoxicity and oxidative stress in animal model of epileptic seizure

Four-week-old male ICR mice were fed with normal diet, tyrosine (L-Tyr), or tyrosine-containing peptide (3xYQ, 1xYW, or 3xYW) for one week. Additionally, an intraperitoneal injection of 200 mg/kg YQ was carried out (YQ i.p). Kainic acid (KA) was dissolved in saline by heating in a water bath, resulting in a 4.5 mg/ml KA solution. The resulting KA solution was then intraperitoneally injected at a dose of 36 mg/kg, and seizure level and symptoms were recorded for 2 hours. The brain tissues (prefrontal cortex and hippocampus) of the mice that survived 16 hours or 5 days after the KA injection were collected.

5-1. Determination of seizure level

Determination of seizure level was determined according to the criteria given in the following Table 2.

[Table 2]

| Key symptoms of each seizure level | |
| --- | --- |
| Level | Key Symptoms |
| I | Sit or stay still in the corner, eyes focused |
| II | Stretched body, tight and rigid tail, ears held back, and bulged red eyes |
| III | Repeated head twitching, sit with front paws on stomach |
| IV | Running and jumping with intermittent seizure, remain calm, sit and lie due to tonic-clonic seizure |
| V | Continuous seizure |
| VI | Intermittent body seizure, not much of continuous body balance using paws, and mostly dead |

The results showed a significant decrease in seizure level in the peptide diet group compared to the normal diet group (FIG. 16).

5-2. Measurement of reactive oxygen species/reactive nitrogen species (ROS/RNS)

The hippocampus was homogenized using 100 $\mu$l of RIPA buffer per 10 mg of tissue weight, which included protease and phosphatase inhibitors. The homogenization was performed using glass beads and blender for 1 minute. The homogenates were then centrifuged at 12,000 $\times$ g for 15 minutes at 4°C, and the supernatant was collected and diluted 10 times in PBS for ROS/RNS measurement. The ROS/RNS concentration was measured using the OxiSelect ROS/RNS Assay Kit (Cell Biolabs) by following the recommended experimental procedures.

The results showed that, in the hippocampal tissue of survived mice after kainic acid injection, there is an increase in ROS/RNS concentration caused by kainic acid. However, the concentration was found to be decreased by the presence of 3xYQ or 3xYW peptide (FIG. 17).

5-3. Measurement of activity of glutamine synthetase (GS)

To a 96-well plate, 2 μl of hippocampal homogenate was added, and 50 μl of 50 mM imidazole-HCl buffer (pH 6.8) were added to adjust the volume to 50 μl. Then, 50 μl of GS assay buffer (50 mM imidazole-HCl, pH 6.8, 25 mM L-glutamine, 12.5 mM hydroxylamine, 12.5 mM sodium arsenate, 1 mM $MnCl_2$, and 0.08 mM ADP) were added and the reaction was allowed to occur at 37°C for approximately 40 minutes. After the reaction, the standard substance γ-glutamylhydroxamate was added to empty wells to have concentration ranging from 0.391 to 25.0 mM. To terminate the reaction, 100 μl of stop solution (90 mM $FeCl_3$, 1.8 N HCl, and 1.45% w/v trichloroacetic acid) were added to both the samples and the standard substance, and the absorbance was measured at 560 nm. The GS activity in each sample was determined by comparing it with the standard curve. GS activity was expressed as the generation amount of γ-glutamylhydroxamate, which is produced as a final product, and described in unit of μM/min/μg protein.

The results showed that, in the hippocampal tissue of survived mice after kainic acid injection, there is a decrease in the activity of the glutamine synthetase caused by kainic acid, but it increased in the presence of 3xYQ, 1xYW, 3xYW, or 3xYQ peptide (FIG. 18).

Example 6. Analysis of inhibitory effect on neuronal cell death and anti-oxidation effect in animal model of ischemic stroke

Three-month-old male Mongolian gerbils were induced with general anesthesia using 3% isoflurane and the anesthesia was maintained with 2.5% isoflurane gas. After sterilizing the neck area, a midline incision was made to expose both common carotid arteries, and microvessel clip was used to block the blood flow for 5 minutes, inducing cerebral ischemia to simulate a stroke. During the application of the microvessel clip, the body temperature was maintained at 37.0 ±0.5°C, and animals undergoing sham operation were used as the control group.

6-1. Analysis of inhibitory effect on neuronal cell death

After inducing ischemia, the animal was, under reperfusion, intraperitoneally administered with YQ peptide at a dose of 200 mg/kg, three times every 24 hours. On the fourth day, the animal was sacrificed, and brain tissues were collected to measure the number of dead neuronal cell.

The results showed a decrease in the number of neuronal cells in the ischemic stroke animal model compared to the sham operation group, but an increase in the neuron number cells was observed with the administration of YQ peptide (FIG. 19A).

6-2. Measurement of activities of SOD and CAT

After inducing ischemia, the animal was, under reperfusion, intraperitoneally administered once with YQ peptide at a dose of 200 mg/kg. Two hours or twenty-four hours after the reperfusion, the animal was anesthetized with avertin. Then, the skull was quickly opened, and the brain was rapidly removed. The hippocampal tissue was collected and rapidly frozen in liquid nitrogen, then stored in an deep freezer. Subsequently, the activities of superoxide dismutase (SOD) and catalase (CAT) were measured by using SOD colorimetric activity kit and CAT colorimetric activity kit, respectively.

The results showed, twenty-four hours after the reperfusion, a decrease in SOD and CAT activities in the ischemic stroke animal model compared to the sham operation group, but activity increases were observed in accordance with the administration of YQ peptide (FIGs. 19B and 19C).

6-3. Immunohistochemical staining for determining neuronal cell death

Immunohistochemical staining was carried out to examine changes in neuronal cells and microglial cells and also a change in related factors in the hippocampus of an ischemic brain injury animal model. Specifically, after inducing ischemia, YQ peptide was administered intraperitoneally three times every 24 hours at a dose of 200 mg/kg under reperfusion. A control group was administered with a vehicle (i.e., 0.9% saline) instead of the peptide. On the 4th day, the animal was sacrificed, and brain tissue was collected and fixed with 4% paraformaldehyde at 4°C. Subsequently, the tissue was added to sucrose solutions of increasing concentrations (i.e., 10%, 20%, and 30%), frozen using liquid nitrogen, and sectioned into continuous slice specimens with a thickness of 30 μm. The specimens were stored in a cryoprotective solution at -20°C. The brain tissue specimen was rinsed three times for 10 minutes each with 0.01 M PBS and treated with 0.3% $H_2O_2$ for 30 minutes to eliminate endogenous peroxidase present in the tissue. To prevent any nonspecific immune reaction, the specimen was incubated for 30 minutes with 5% normal serum corresponding to each antibody. Then, an antibody related to anti-cone-shaped neuronal cell (NeuN), an antibody related to anti-star-like glial cell (GFAP), and an antibody related to anti-microglial cell (Iba-1) were diluted at dilution factor for each and incubated overnight at room temperature with the specimen. After the completion of the incubation, the tissue specimen was incubated for 2 hours with a biotin-conjugated secondary antibody followed by a reaction in ABC solution for 1 hour. Upon the completion of the reaction, the tissue specimen was developed using a 3,3'-DAB kit to exhibit the color, and then mounted on a glass slide and dried for 12 hours at room temperature. Subsequently, the specimen was subjected to the typical dehydration and clearing process, followed by sealing using DPX mounting solution. It was then observed under a microscope (Olympus BX53).

As a result, in the control group (0.9% saline-treated group), very few surviving neurons were observed in the hippocampal CA1 region. However, in the group administered with YQ peptide, approximately 60.7% of cells were found to survive compared to the sham operation group. Additionally, in the ischemic brain injury animal model, the control group (0.9% saline-treated group) showed glial cells with enlarged cell cytoplasm and enlarged cell spikes. In contrast, the group administered with YQ peptide exhibited minimal morphological changes in glial cells, closely resembling the morphology of the sham operation group (FIG. 20).

Based on these findings, it is believed that the peptide containing terminal tyrosine protects against neuronal cell death by suppressing oxidative damage and immune responses in ischemic brain tissues.

Example 7. Analysis of effect on insulin sensitivity enhancement using high-fat diet-induced type 2 diabetic animal model

Three-week-old male C57BL/6 mice were purchased from Koatech and housed in the animal facility under controlled conditions of temperature ($22\pm2°C$), humidity ($50\pm5\%$), and a 12-hour light-dark cycle. Two mice were housed per a cage. After one-week acclimation period, the mice were divided into four groups: normal diet group (ND) receiving a standard diet, high-fat diet group (HFD) receiving a diet containing 60% of calories from fat, and high-fat diet groups with YQ peptide supplementation, i.e., HFD with 1xYQ peptide supplementation (HFD+1xYQ) and HFD with 3xYQ peptide supplementation (HFD+3xYQ). The mice were maintained on their respective diets for a duration of 17 weeks.

7-1. Measurement of body weight, feed intake amount, and fasting blood glucose level

With an interval of one week, the body weight and food intake of each animal were measured. Additionally, at 1, 5, or 8 weeks after the start of the experiment, fasting blood glucose level was measured using an Accu-Check glucose meter.

The results showed that the body weight gradually increased in all test groups. On the same day, the HFD group was found to have higher body weight compared to the ND group while the HFD+3xYQ group had lower body weight than the HFD group (FIG. 21A). On the same day, the food intake was lower in the HFD, HFD+1xYQ, and HFD+3xYQ groups compared to the ND group, but there were no significant differences among the HFD, HFD+1xYQ, and HFD+3xYQ groups (FIG. 21B).

Furthermore, the blood glucose level in the HFD group was higher than the level in the ND group, while the HFD+1xYQ and HFD+3xYQ groups showed a decrease in blood glucose level over time compared to the HFD group (FIG. 22).

7-2. Glucose tolerance test (GTT) and insulin tolerance test (ITT)

At 15 weeks after the start of the test, a glucose tolerance test (GTT) was performed, and at 16 weeks, an insulin tolerance test (ITT) was carried out. For the GTT, after a 12-hour fasting period, a glucose solution of 2 g/kg was administered through intraperitoneal injection, and blood samples were collected from the vein at 0, 20, 60, 90, and 120 minutes to measure blood glucose level. For the ITT, after a 6-hour fasting period, insulin at a dose of 0.75 U/kg was administered through intraperitoneal injection, and blood samples were collected from the vein at 0, 15, 30, 60, and 120 minutes to measure blood glucose level.

From the result of measuring the glucose tolerance and insulin sensitivity, it was found that the HFD group had increased blood glucose level compared to the ND group. On the other hand, the HFD+3xYQ group showed a decrease in blood glucose level compared to the HFD group (FIGs. 23 and 24).

7-3. Measurement of urine volume and fat mass

The urine volume (ml) was measured by collecting urine from the metabolic cages over a 16-hour period, and the fat mass was measured for each mouse using the EchoMRI™ device and expressed as a percentage (%) of body weight.

The results showed that the HFD group had higher urine volume and higher fat mass compared to the ND group. On the other hand, the HFD+3xYQ group exhibited a decrease in urine volume and fat mass compared to the HFD group (FIG. 25).

7-4. Biochemical analyses of blood plasma

After anesthesia, blood was collected from the inferior vena caba, and centrifuged at 3,000 rpm for 15 minutes to separate the plasma. The insulin concentration was measured using an ELISA kit (Crystal Chem, Ultra Sensitive Mouse Insulin ELISA Kit), and the alanine aminotransferase (ALT) concentration was measured using an assay kit (IVD Lab Co., ChemiLab GPT (ALT) assay kit). Additionally, the concentration of reactive oxygen species/reactive nitrogen species (ROS/RNS) was measured by using an assay kit (Cell Biolabs, Inc., OxiSelect™ In Vitro ROS/RNS Assay Kit).

As a result, it was observed that compared to the ND group, the HFD group exhibited increased concentrations of insulin, ALT, and ROS/RNS in the plasma. However, compared to the HFD group, the HFD+3xYQ group showed decreased concentrations of insulin, ALT, and ROS/RNS in the plasma (FIG. 26).

Based on these findings, it can be concluded that the peptide with terminal tyrosine could be used for the prevention or treatment of non-alcoholic liver disease and chronic metabolic disorders caused by reactive oxygen species/reactive nitrogen species.

7-5. Histological analysis

After anesthesia, liver tissues were dissected and rapidly frozen in liquid nitrogen. Some tissues were fixed in 10% formalin, and 5 $\mu$m-thick tissue slides were prepared from paraffin blocks. The tissues were then stained with H&E (hematoxylin and eosin) and observed under a microscope (Olympus, CKX41) for imaging.

The results showed that the liver in the HFD group exhibited traces of lipid droplets in liver cells and tissue fibrosis, while the HFD+3xYQ group maintained a liver tissue appearance similar to the ND group (FIG. 27).

7-6. Measurement of expression level of insulin receptor

A decrease in insulin receptor in muscles in metabolic disorders reduces the sensitivity to insulin signal, thus limiting the ability to utilize glucose in the muscles to cause sarcopenia. The liver tissue was homogenized in RIPA buffer, and protein quantification was performed using the BCA method. Western blot analysis was then conducted using an anti-insulin receptor-beta (IR$\beta$) antibody.

As a result, a decrease in the expression level of insulin receptor-beta (IR$\beta$) was observed in the HFD group compared to the ND group, while an increase in the expression level of IR$\beta$ was observed in the HFD+3xYQ group compared to the HFD group (FIG. 28).

Based on these results, it can be concluded that the peptide containing terminal tyrosine increases the expression level of insulin receptor-beta, thereby exhibiting a preventive effect against sarcopenia associated with metabolic disorders.

7-7. Measurement of albumin, creatinine, and triglyceride levels

It is known that a decrease in renal function leads to an increase in the concentration of albumin and a decrease in creatinine in urine. Therefore, the albumin and creatinine levels in urine were measured using mouse albumin ELISA kit (Abcam) and creatinine assay kit (Abcam), respectively, in each test animal. The albumin/creatinine ratio was then calculated. Additionally, 10 $\mu$L of blood plasma or 40 mg of homogenized liver tissue were subjected to centrifugation at 10,000 g for 10 minutes, and the supernatant was separated to measure the triglycerides (TG) level in plasma or liver by using a triglyceride measurement kit (Cayman).

The results showed that compared to the ND group, the HFD group had increased albumin/creatinine ratio and triglycerides level. In contrast, compared to the HFD group, the HFD+1xYQ or HFD+3xYQ group showed decreased albumin/creatinine ratio and decreased triglycerides level (FIG. 29).

Based on these results, it is found that the peptide with terminal tyrosine has an effect of inhibiting renal function decline caused by diabetes, thereby preventing diabetic nephropathy, which is one of the complications of diabetes. Additionally, it suggests a preventive or therapeutic effect on dyslipidemia, which is characterized by abnormal increase in blood triglyceride level.

Example 8. Analysis of inhibitory effect on protein nitration in animal model of acute renal failure caused by renal ischemia reperfusion

Male C57BL/6 mice weighing 23 to 25 g were housed in a germ-free facility with controlled temperature and humidity. They were allowed free access to water and food. The mice were divided into three groups: 1) Control group (Sham), 2) induced renal ischemia-reperfusion group (IR) administered with water (Veh+renal IR), and 3) induced renal ischemia-reperfusion group administered with YQ peptide (YQ+renal IR). YQ peptide (100 mg/kg) was administered orally, once a day for four days, and on the fourth day, renal ischemia was induced 30 minutes after the last oral administration. Renal ischemia was induced by midline laparotomy and occlusion of both renal pedicles using a Müller atraumatic vascular clamp for 25 minutes, followed by removal of the clamp for reperfusion. Control group (sham) underwent the same surgical procedures except the induction of ischemia using clamp. After 24 hours of reperfusion, the mice were sacrificed, and blood was collected from the heart, and the kidney tissue was harvested.

8-1. Measurement of blood creatinine level

The collected blood was centrifuged at 3,000 rpm for 15 minutes to separate the serum, and the serum creatinine level, an indicator for evaluating kidney damage, was measured using the Jaffe method. The Jaffe method involves measuring the absorbance of the serum sample at 510 nm wavelength after reacting it with picric acid and then terminating the reaction with 60% acetic acid followed by measurement of absorbance, and calculating the concentration of creatinine in mg/mL units based on the difference in absorbance.

The results showed a significant increase in plasma creatinine level after 24 hours of renal ischemia and reperfusion compared to the control group (sham). However, this increase was statistically significantly suppressed by the administration of YQ peptide (FIG. 30A).

8-2. Real-time qPCR analysis

The total RNA was extracted from the kidney tissues using the Trizol method, and cDNA was synthesized using the RevertAid Reverse Transcription System (ThermoFisher). Quantitative PCR (qPCR) for inflammatory cytokines (IL-1β, IL-6, and MCP-1) was performed using the iQ SYBR Green Supermix (Bio-Rad) on the CFX Connect Real-Time PCR system (Bio-Rad). The relative amount of the target mRNA, normalized to GAPDH, was analyzed using the $2^{-\Delta Ct}$ method. The primers used in the experiment are listed in Table 3.

[Table 3]

| Information of primers used for qPCR analysis | | |
|---|---|---|
| Gene | Primer sequence (5'-3') | SEQ ID NO: |
| IL-1β | F: TCGCAGCAGCACATCAACAAGAG | 1 |
| | R: GGTGCTCATGTCCTCATCCTGGA | 2 |
| IL-6 | F: CCAATTCATCTTGAAATCAC | 3 |
| | R: GGAATGTCCACAAACTGATA | 4 |
| MCP-1 | F: ACCTTTGAATGTGAAGTTGA | 5 |
| | R: CTACAGAAGTGCTTGAGGTG | 6 |
| GAPDH | F: GTGGCAAAGTGGAGATTGTTG | 7 |
| | R: TTGACTGTGCCGTTGAATTTG | 8 |

The results showed that the expression level of IL-1β, IL-6, and MCP-1, which are the indicators of the inflammatory response as a main onset mechanism of acute renal failure, was significantly increased after 24 hours of renal ischemia and reperfusion compared to the control group (sham). However, it was also found that this increase was statistically significantly inhibited by the administration of YQ peptide (FIGs. 30B, 30C, and 30D).

8-3. Measurement of the levels of nitrated proteins and lipid peroxidation in kidney tissue

The kidney tissue was homogenized in RIPA buffer, and protein quantification was performed using the BCA method. Western blot was then carried out using antibodies against nitrotyrosine and 4-hydroxynonenal (4-HNE), i.e., a product of lipid peroxidation.

The results showed that the proteins with nitrated tyrosine and lipid peroxidation in the kidney tissue increased after 24 hours of renal ischemia and reperfusion compared to the control group (sham). However, the increases were significantly reduced by the administration of YQ peptide (FIG. 31).

Example 9. Analysis of inhibitory effect on blood ammonia in animal model of hyperammonemia

Male C57BL/6 mice at 13 weeks of age were housed in a germ-free facility with controlled temperature and humidity. They were allowed free access to water and food. The mice were divided into four groups: (1) control group (Control), (2) hyperammonemia group administered with water (Veh+AOM), (3) hyperammonemia group administered with 100 mg/kg YQ peptide (YQ100+AOM), and (4) hyperammonemia group administered with 200 mg/kg YQ peptide (YQ200+AOM). YQ peptide was administered orally once a day for four consecutive days. On the fourth day, 2 hours after the last oral administration of YQ peptide, azoxymethane (AOM) at a dose of 100 mg/kg was intraperitoneally injected to induce hyperammonemia. To prevent dehydration 12 hours after AOM administration, 200 $\mu$l of physiological saline containing 0.5% glucose were injected into the abdominal cavity. After 4 hours, the mice were sacrificed, and blood was collected from the heart, and liver tissue was collected.

9-1. Analysis of blood ammonia

Ammonia is a primary metabolite of amino acids and nucleic acids, and the main enzymes involved in the conversion of ammonia into urea in urea cycle are found only in liver cells. In addition, the liver tissue is also rich in enzymes such as GS (glutamine synthetase) and SOD (superoxide dismutase), which help lower ammonia level, and catalase which removes reactive oxygen species. Therefore, a change in blood ammonia level as it travels from the liver to the brain through the bloodstream serves as an important indicator of hepatic encephalopathy. Accordingly, to measure the amount of blood ammonia, a PocketChem BA PA-4140 analyzer (Arkray, Japan), which is a single-wavelength reflectance measurement analyzer, was utilized. The analyzer measures the absorbance at 635 nm (LED) wavelength after the reaction 20 $\mu$l of blood with the test strip at room temperature for 3 minutes. The results are then displayed in $\mu$g/dL units.
The results showed that the hyperammonemia group (Veh+AOM), which received water, had approximately a 3.6-fold increase in blood ammonia level compared to the control group (control). On the other hand, it was also observed that the administration of YQ peptide significantly suppressed the increase in blood ammonia caused by AOM (FIG. 32A).

9-2. Expression level of nitrated proteins in liver tissue

The liver tissue was homogenized in RIPA buffer, and protein content was quantified using the BCA method. Western blot analysis was performed using an antibody against nitrotyrosine.
The results showed an increase in nitrotyrosine protein level in the liver tissue after AOM administration compared to the control group. However, this increase was significantly reduced by the administration of YQ peptide. These results indicate that YQ peptide can inhibit protein nitration in the liver, thereby facilitating the removal of ammonia in the liver (FIG. 32B).

[Statistical Processing]

All data of the present invention are presented as mean $\pm$ standard deviation, and were statistically analyzed using Dunnett's multiple comparison test for one-way analysis of variance (ANOVA) or Student's t-test using GraphPad Prism 5 software.

EMBODIMENTS OF THE DISCLOSURE

Embodiment 1. A functional health food composition for preventing or ameliorating a disease caused by protein nitration comprising a peptide with terminal tyrosine or a salt thereof, which is acceptable for use in food, as effective component.
Embodiment 2. The functional health food composition according to Claim 1, wherein the peptide with terminal tyrosine can have tyrosine positioned at both terminals or at one terminal, and an amino acid connected to the tyrosine ranges from 1 to 29 amino acids.
Embodiment 3. The functional health food composition according to Claim 1, wherein the protein is any one selected from glutamine synthetase, insulin receptor $\beta$ subunit, Mn superoxide dismutase, heat shock protein 60, Cu/Zn superoxide dismutase, and catalase.
Embodiment 4. The functional health food composition according to Claim 1, wherein the disease caused by protein nitration is any one selected from depressive disorder, anxiety disorder, stroke, epilepsy, seizure, cognitive impairment, Alzheimer's disease, dementia, type 2 diabetes, diabetic nephropathy, sarcopenia, dyslipidemia, obesity, non-alcoholic fatty liver disease, acute kidney injury, hyperammonemia, and hepatic encephalopathy.
Embodiment 5. A pharmaceutical composition for preventing or treating a disease caused by protein nitration comprising a peptide with terminal tyrosine or a pharmaceutically acceptable salt thereof as effective component.

Embodiment 6. A composition for inhibiting tyrosine nitration in protein comprising a peptide with terminal tyrosine or a pharmaceutically acceptable salt thereof as effective component.

Embodiment 7. A method for removing a nitro group from nitrotyrosine by treating a protein having nitrotyrosine with a peptide with terminal tyrosine.

Embodiment 8. A functional health food composition for preventing or ameliorating a disease caused by an increase in reactive oxygen species/reactive nitrogen species comprising a peptide with terminal tyrosine or a salt thereof, which is acceptable for use in food, as effective component.

Embodiment 9. A pharmaceutical composition for preventing or treating a disease caused by an increase in reactive oxygen species/reactive nitrogen species comprising a peptide with terminal tyrosine or a pharmaceutically acceptable salt thereof as effective component.

Embodiment 10. The pharmaceutical composition according to Claim 9, wherein the disease caused by an increase in reactive oxygen species/reactive nitrogen species is any one selected from depressive disorder, anxiety disorder, stroke, epilepsy, seizure, cognitive impairment, Alzheimer's disease, dementia, type 2 diabetes, diabetic nephropathy, sarcopenia, dyslipidemia, obesity, non-alcoholic fatty liver disease, acute kidney injury, hyperammonemia, and hepatic encephalopathy.

## Claims

1. A composition for preventing, ameliorating or treating a disease caused by protein nitration comprising a peptide with terminal tyrosine or a salt thereof.

2. The composition according to Claim 1, wherein the peptide with terminal tyrosine can have tyrosine positioned at both terminal or at one terminal, and an amino acid connected to the tyrosine ranges from 1 to 29 amino acids.

3. The composition according to Claim 1, wherein the protein any one selected from the group consisting of glutamine synthase, insulin receptor β, Mn superoxide dismutase, heat shock protein 60, Cu/Zn superoxide dismutase and catalase.

4. The composition according to Claim 1, wherein the disease caused by protein nitration is any one selected from the group consisting of depressive disorder, anxiety disorder, stroke, epilepsy, seizure, cognitive impairment, Alzheimer's disease, dementia, type 2 diabetes, diabetic nephropathy, sarcopenia, dyslipidemia, obesity, non-alcoholic fatty liver disease, acute kidney injury, hyperammonemia, and hepatic encephalopathy.

5. The composition according to Claim 1, wherein the composition reduces reactive oxygen species (ROS), or reactive nitrogen species (RNS), or oxygen species (ROS) and reactive nitrogen species (RNS).

6. The composition according to Claim 1, wherein the composition removes the nitro group from nitrotyrosine in a protein with a tyrosine moiety.

7. The composition according to Claim 1, wherein the composition is produced in any formulation selected from the group consisting of a pill, a tablet, a powder, a granule, a syrup, a candy and a drink.

8. The composition according to Claim 1, wherein the composition further comprises a carrier, a vehicle or a diluent.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

A

B

[FIG. 16]

[FIG. 17]

[FIG. 18]

[FIG. 19]

[FIG. 20]

[FIG. 21]

[FIG. 22]

[FIG. 23]

[FIG. 24]

A — ITT

B — ITT (AUC)

[FIG. 25]

A — Urine volume (ml)

B — Fat mass

[FIG. 26]

[FIG. 27]

[FIG. 28]

[FIG. 29]

[FIG. 30]

[FIG. 31]

[FIG. 32]

A

B

**REFERENCES CITED IN THE DESCRIPTION**

**Patent documents cited in the description**

- KR 1897400

- KR 20180021746